(19) Europäisches Patentamt
European Patent Office
Office européen des brevets

(11) Veröffentlichungsnummer: **0 399 267 A2**

(12) **EUROPÄISCHE PATENTANMELDUNG**

(21) Anmeldenummer: **90108496.2**

(22) Anmeldetag: **07.05.90**

(51) Int. Cl.5: **C07D 409/12, C07D 405/12, C07D 401/12, A61K 31/47**

(30) Priorität: **26.05.89 DE 3917233**

(43) Veröffentlichungstag der Anmeldung: **28.11.90 Patentblatt 90/48**

(84) Benannte Vertragsstaaten: **AT BE CH DE DK ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Geiss, Karl-Heinz, Dr.**
**Eckbachring 46**
**D-6711 Heuchelheim(DE)**
Erfinder: **Ruebsamen, Klaus, Dr.**
**Kaiserstuhl 6**
**D-6730 Neustadt(DE)**
Erfinder: **Traut, Martin, Dr.**
**Muehltalstrasse 125**
**D-6900 Heidelberg(DE)**

(54) **8-Substituierte 4-(Heterocyclylmethylamino)-chinoline, ihre Verwendung und daraus hergestellte Arzneimittel.**

(57) 8-Substituierte 4-(Heterocyclylmethylamino)-chinoline der Formel I

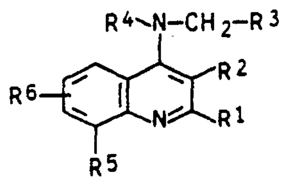

(I),

worin die Reste $R^1$-$R^6$ die in den Ansprüchen genannten Bedeutungen besitzen, sowie deren physiologisch verträgliche Salze, ihre Verwendung zur Herstellung von Arzneimitteln und die so erhältlichen Arzneimittel, insbesondere gegen Krankheiten, bei denen eine Herabsetzung der Magensäuresekretion die Heilung günstig beeinflußt.

EP 0 399 267 A2

## 8-Substituierte 4-(Heterocyclylmethylamino)-chinoline, ihre Verwendung und daraus hergestellte Arzneimittel

Die Erfindung betrifft neue 8-substituierte 4-(Heterocyclylmethylamino)-chinoline sowie deren Verwendung zur Bekämpfung von Krankheiten.

In allgemeiner Form wurden 2-Alkyl-4-aralkylamino-chinoline als Zwischenprodukte in der US-P 3 075 984 genannt; für diese Zwischenstufen wurden keine pharmakologischen Wirkungen beschrieben. 4-Heteroarylmethylaminochinoline mit fungizider Wirksamkeit wurden in der US 4 744 823 beschrieben.

Aus dem weiteren Umfeld pharmakologisch aktiver 4-Aminochinoline mit stark variiertem Substitutionsmuster am Chinolingerüst sind zu nennen:

In 2-Position unsubstituierte 4-Anilino- und 4-Benzylaminochinolin-3-yl-ketone und -carbonsäureester mit magensäuresekretionshemmender Wirkung (US-P 4 343 804, US-4 806 549 und US 4 806 550).

4-Amino-2-methylchinolin-3-yl-ketone und -alkanole zur Behandlung von Morbus Alzheimer (US 4 789 678 und 4 840 970).

In der nicht vorveröffentlichten Patentanmeldung DE 3 908 767.0 werden 2-Alkyl-4-benzylamino-8-methoxychinoline beschrieben, die Hemmstoffe der $K^+/H^+$-ATPase darstellen.

Es wurde nun gefunden, daß die erfindungsgemäßen Verbindungen der Formel I

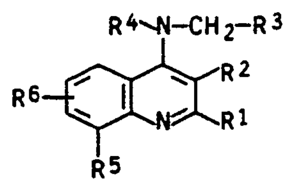

(I),

in der

$R^1$ ein Wasserstoffatom oder eine $C_1$-$C_4$-Alkylgruppe, die durch eine Hydroxi- oder eine $C_1$-$C_4$-Alkoxigruppe substituiert sein kann,

$R^2$ ein Wasserstoffatom oder eine $C_1$-$C_6$-Alkylgruppe,

$R^3$ einen Thienylrest, der durch 1 bis 3 $C_1$-$C_4$-Alkylgruppen und/oder 1 bis 2 Halogenatome substituiert sein kann,

einen Furanylrest, der durch 1 bis 3 $C_1$-$C_4$-Alkylgruppen substituiert sein kann,

einen Pyrrol-yl-rest, der am Pyrrol-Stickstoffatom ein Wasserstoff, eine $C_1$-$C_6$-Alkylgruppe, einen Phenyl($C_1$-$C_3$-alkyl)rest oder einen Phenylrest tragen kann und an den Kohlenstoffatomen des Pyrrol-Ringes mit 1 bis 3 $C_1$-$C_4$-Alkylgruppen substituiert sein kann,

wobei der Thienyl-, Furanyl- oder Pyrrolylrest auch benzokondensiert sein kann,

$R^4$ ein Wasserstoffatom, eine $C_1$-$C_4$-Alkylgruppe oder den Rest $-CH_2-R^3$, wobei $R^3$ obige Bedeutung besitzt,

$R^5$ eine $C_1$-$C_6$-Alkylgruppe, die durch eine Hydroxi- oder $C_1$-$C_3$-Alkoxigruppe substituiert sein kann, eine Hydroxi-, $C_1$-$C_6$-Alkoxi-, Phenoxi-, Phenyl-($C_1$-$C_3$-alkoxi)-, $C_1$-$C_6$-Alkylthio-, Phenylthio-, Phenyl-($C_1$-$C_3$-alkylthio)-, Amino-, $C_1$-$C_4$-Alkylamino-, Di-($C_1$-$C_4$-alkyl)amino-, Phenylamino-, N-Phenyl-N-($C_1$-$C_4$-alkyl)-amino-, (Phenyl-($C_1$-$C_4$-alkyl))amino-, N-(Phenyl($C_1$-$C_4$-alkyl))-N-($C_1$-$C_4$-alkyl)-amino-, $C_1$-$C_4$-Alkanoylamino-, Benzoylamino-, (Phenyl-($C_1$-$C_3$-alkanoyl))amino-, $C_1$-$C_6$-Alkanoyl-, Hydroxycarbonyl-, $C_1$-$C_6$-Alkoxicarbonyl-, Aminocarbonyl-, $C_1$-$C_6$-Alkylaminocarbonyl-, Di-($C_1$-$C_6$-Alkyl)amino-carbonyl- oder Trifluormethylgruppe oder ein Halogenatom, und

$R^6$ ein Wasserstoffatom, eine $C_1$-$C_4$-Alkyl- oder $C_1$-$C_4$-Alkoxygruppe oder ein Halogenatom

bedeuten, sowie deren physiologisch verträgliche Salze wertvolle pharmakologische Wirkungen besitzen.

Bevorzugte Bedeutungen von $R^1$ sind ein Wasserstoffatom und eine $C_1$-$C_3$-Alkylgruppe, die durch eine Hydroxi- oder $C_1$-$C_3$-Alkoxigruppe substituiert sein kann. Bedeutet $R^1$ eine $C_1$-$C_3$-Alkylgruppe, so ist eine Methylgruppe, die durch eine Hydroxy-oder $C_1$-$C_3$-Alkoxygruppe, insbesondere die Methoxigruppe, substituiert sein kann, besonders bevorzugt.

Bevorzugte Bedeutungen von $R^2$ sind ein Wasserstoffatom oder eine $C_1$-$C_3$-Alkylgruppe.

In einer bevorzugten Gruppe der erfindungsgemäßen Verbindungen bedeuten $R^1$ und $R^2$ jeweils ein Wasserstoffatom.

In einer weiteren bevorzugten Gruppe bedeutet $R^2$ eine $C_1$-$C_3$-Alkylgruppe und $R^1$ ein Wasserstoffatom oder eine $C_1$-$C_3$-Alkylgruppe, die durch eine Hydroxy- oder $C_1$-$C_3$-Alkoxigruppe substituiert sein kann. Wenn $R^2$ eine $C_1$-$C_3$-Alkylgruppe bedeutet, ist eine besonders bevorzugte Bedeutung für $R^1$ ein Wasser-

stoffatom und eine unsubstituierte $C_1$-$C_3$-Alkylgruppe, insbesondere die Methylgruppe.

In einer besonders bevorzugten Gruppe der erfindungsgemäßen Verbindungen bedeutet $R^2$ ein Wasserstoffatom und $R^1$ eine $C_1$-$C_3$-Alkylgruppe, die durch eine Hydroxy-oder $C_1$-$C_3$-Alkoxygruppe substituiert sein kann.

$R^3$ bedeutet bevorzugt einen ggf. substituierten und/oder benzokondensierten Thienyl- oder Pyrrolylrest, insbesondere einen ggf. substituierten und/oder benzokondensierten Thienylrest.

Bevorzugt ist der Furanyl-, Thienyl- oder Pyrrolylrest ein- bis dreifach substituiert.

Bevorzugt steht ein Substituent in $R^3$ in ortho-Stellung zur Verknüpfungsstelle mit der Chinolinaminomethylgruppe.

Bedeutet der Rest $R^3$ einen Thienylrest, der durch 1 bis 3 $C_1$-$C_4$-Alkylgruppen und/oder 1 bis 2 Halogenatome substituiert ist, so ist der Thienylrest vorzugsweise mit 1 bis 3 $C_1$-$C_3$-Alkylgruppen, insbesondere Methylgruppen,und/oder 1 bis 2 Halogenatomen substituiert.

Als Halogene sind Fluor, Chlor, Brom und Jod zu nennen; bevorzugt sind Chlor und Brom.

Bedeutet der Rest $R^3$ einen durch 1 bis 3 $C_1$-$C_4$-Alkylgruppen substituierten Furanylrest, so ist dieser vorzugsweise mit 1 bis 3 $C_1$-$C_3$-Alkylgruppen, insbesondere Methylgruppen, substituiert.

Bedeutet der Rest $R^3$ einen substituierten Pyrrolrest, so sind folgende Substituenten am Pyrrol-Stickstoffatom bevorzugt: eine $C_1$-$C_4$-Alkyl-, eine Benzyl- oder Phenylgruppe, insbesondere eine $C_1$-$C_4$-Alkylgruppe. Als bevorzugte Substituenten an den Kohlenstoffatomen des Pyrrolrings sind 1 bis 3 Methylgruppen zu nennen.

$R^4$ bedeutet bevorzugt ein Wasserstoffatom oder eine Methylgruppe, insbesondere ein Wasserstoffatom.

Bedeutet $R^5$ ein Halogenatom, so sind die Bedeutungen Fluor, Chlor oder Brom zu nennen. Bevorzugt bedeutet $R^5$ eine $C_1$-$C_3$-Alkylgruppe, die durch eine Hydroxi- oder Methoxigruppe substituiert sein kann, eine Hydroxigruppe oder eine $C_1$-$C_3$-Alkoxigruppe, insbesondere steht $R^5$ für eine $C_1$-$C_3$-Alkoxi- oder $C_1$-$C_3$-Alkylgruppe, wobei die Methoxi- und die Methylgruppe besonders bevorzugt sind.

$R^6$ bedeutet bevorzugt eine Methyl-, eine Methoxi-, ein Fluor-, Chlor- oder Bromatom, insbesondere ein Wasserstoffatom.

Die Herstellung der erfindungsgemäßen Verbindungen erfolgt, indem man ein Chinolin der Formel II

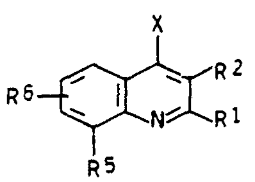

(II),

wobei X eine nukleofuge Abgangsgruppe wie ein Chlor- oder Bromatom oder eine Phenoxygruppe darstellt und $R^1$, $R^2$, $R^5$ und $R^6$ obige Bedeutungen besitzen, und wobei, falls $R^1$ oder $R^5$ eine durch eine Hydroxygruppe substituierte $C_1$-$C_4$-Alkylgruppe oder/und $R^5$ eine primäre oder sekundäre Aminogruppe bedeutet, die Hydroxy- bzw. die Aminogruppe durch eine geeignete übliche Schutzgruppe modifiziert werden kann, mit einem Amin der Formel $R^3$-$CH_2$-NH-$R^4$, wobei $R^3$ und $R^4$ obige Bedeutungen besitzen, in üblicher Weise umsetzt und die Hydroxy- oder Amino-Schutzgruppe, falls vorhanden, wieder abspaltet.

Die Reaktion kann in Gegenwart eines Lösungsmittels wie Toluol, Xylol, Phenol, Ethanol, Butanol, Dimethylsulfoxid, Dimethylethylenharnstoff, Dimethylpropylenharnstoff, Pyrrolidon, N-Methylpyrrolidon, in Gemischen dieser Lösungsmittel oder in Abwesenheit von Lösungsmitteln, gegebenenfalls in Gegenwart eines Katalysators wie Kupfer- oder Bronzepulver oder Kupfer(I)-chlorid, bei Temperaturen zwischen 50 und 250 °C, gegebenenfalls unter Druck, durchgeführt werden. Die Amine $R^3CH_2NHR^4$ können in äquimolekularen Mengen oder im Überschuß eingesetzt werden.

Bevorzugt wird die Umsetzung der Verbindungen der Formel II mit den Aminen $R^3CH_2NHR^4$ im Verhältnis 1:1 bis 1:10 in Gegenwart von Phenol bei Temperaturen von 60 bis 160 °C.

Die Herstellung von 4-Aminochinolinen nach obigem Verfahren wurde u. a. in folgenden Literaturstellen beschrieben:

G. Jones, Quinolines, Part I, John Wiley & Sons, London, New York, 1977, S. 547-550 u. dort zit. Lit.; J. Indian Chem. Soc. 51 (1974) 880-882; J. Med. Chem. 14 (1971) 1060-1066; Chim. Therap. 1 (1966) 339-346; Eur. J. Med. Chem 11 (1976) 561-565.

Die Verbindungen der allgemeinen Formel II können analog bekannten Verbindungen erhalten werden. Zur Darstellung der Verbindungen der Formel II siehe z.B. J. Indian Chem. Soc. 51 (1974), 880-882 sowie G. Jones (Ed) Quinolines, Part I, John Wiley & Sons, London, 1977: X = Cl: S. 391-398, X = Br: S. 404-

406, X = $OC_6H_5$: S. 577-579. 4-Phenoxychinoline können auch bei der Umsetzung der 4-Chlorchinoline mit Aminen $R^3CH_2NHR^4$ in Gegenwart von Phenol als Zwischenstufen nachgewiesen werden.

Die Amine $R^3CH_2NHR^4$ sind literaturbekannt, bzw. lassen sich analog bekannten Verbindungen herstellen (Houben Weyl, Methoden der Organischen Chemie Bd. 11/1, 4. Auflage 1957, G. Thieme-Verlag Stuttgart).

Gegebenenfalls werden die erhaltenen erfindungsgemäßen Verbindungen in das Säureadditionssalz einer physiologisch verträglichen Säure überführt. Eine Zusammenstellung üblicher physiologisch verträglicher Säuren kann aus Fortschritte der Arzneimittelforschung 1966, Deutschland, Schweiz, Birkhäuser Verlag, Bd. 10, S. 224-285 und J. Pharm. Sci., Bd. 66 (1977), S. 1-5 entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether, wie Diethylether, Tetrahydrofuran oder Dioxan erhalten. Zur besseren Kristallabscheidung können Mischungen der genannten Lösungsmittel verwendet werden. Darüber hinaus können pharmazeutisch vertretbare wäßrige Lösungen von Säure-Additionsverbindungen der Aminoverbindungen der Formel I durch Auflösen der freien Basen in einer wäßrigen Säurelösung hergestellt werden.

Die erfindungsgemäßen Verbindungen und ihre Salze mit physiologisch verträglichen Säuren besitzen wertvolle pharmakologische Wirkungen. Insbesondere hemmen sie die gastrointestinale $K^+ H^+$-ATPase und die Magensäuresekretion. Die erfindungsgemäßen Verbindungen können daher zur Therapie aller Krankheiten, bei denen eine Herabsetzung der Magensäuresekretion die Heilung günstig beeinflußt, z. B. Ulcus ventriculi, Ulcus duodeni, Gastritis, Reflux-Ösophagitis, Zollinger-Ellison-Syndrom eingesetzt werden (vgl. Review über Hemmstoffe der $K^+ H^+$-ATPase, G. Sachs et al., Ann. Rev. Pharmacol. Toxicol. 28 (88) 269-284 und dort zit. Lit.).

Gegenstand der vorliegenden Erfindung sind auch Arzneimittel zur oralen, rektalen oder intravenösen Applikation, die neben üblichen Träger- und Verdünnungsmitteln die Verbindungen der Formel I oder deren Säureadditionssalze als Wirkstoff enthalten, sowie die Verwendung der neuen Verbindungen und ihrer physiologisch verträglichen Salze bei der Behandlung der genannten Krankheiten.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen. Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und oder Mitteln zur Erzielung des Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt, enthalten. Sie können außerdem Suspendierhilfsmittel wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. deren Derivaten, herstellen.

Die Einzeldosierung liegt beim Menschen für die orale oder rektale Anwendung zwischen 10 und 1000 mg und für die i. v. Anwendung zwischen 0,01 und 1,0 mg/kg Körpergewicht.

Die Wirkung der erfindungsgemäßen Verbindungen wurde in der folgenden Testanordnung bestimmt:

Die Mukosa eines frisch entnommenen Schweinemagens wurde im Eisbad in 0,25 M Saccharose, 20 mM Tris (Trishydroxymethyl-amino- methan), 1 mM EGTA (Ethylenbis(oxyethylennitrilo)-tetra- essigsäure) pH 7,0 homogenisiert und 20 min bei 20 000 xg zentrifugiert. Der Überstand wurde 60 min bei 100 000 xg zentrifugiert. Das erhaltene mikrosomale Pellet wurde mit 50 mM Tris + 2 mM $MgCl_2$ + 0,1 mM EGTA, pH 7,5, homogenisiert und bei -20 °C portionsweise eingefroren. Die $K^+/H^+$-ATPase-Aktivität wurde in 1 ml-Ansätzen mit folgender Zusammensetzung getestet: 50 mM Tris/HCl-Puffer, pH 7,5, 2 mM $MgCl_2$, 20 µg Membran-Protein mit oder ohne Zugabe von 5 mM KCl. Die ATPase-Reaktion wurde gestartet durch Zugabe von $Na_2ATP$, Endkonzentration 2 mM, Reaktionszeit 15 min bei 37 °C. Danach wurde die Reaktion durch Zugabe von 1 ml 20 % Trichloressigsäure gestoppt. Die Bestimmung des freigesetzten Phosphats erfolgte nach der Methode von Sanui (Analyt. Biochem. 60, 1974, 489-504).

Durch Zugabe der erfindungsgemäßen Verbindungen in verschiedenen Konzentrationen zur obigen Testanordnung wird der $IC_{50}$-Wert der Hemmung der $K^+/H^+$-ATPase bestimmt.

## Beispiele

Allgemeine Arbeitsvorschrift:

Eine Mischung aus 1 Äquivalent einer Verbindung der Formel II, 1 bis 11 Äquivalenten eines Amins der Formel $R^3CH_2NHR^4$ und 5 bis 20 Äquivalenten Phenol wurde 2 bis 8 h bei 60 bis 140 °C unter Rückfluß oder im Autoklav erhitzt. Nach Abdestillation des überschüssigen Amins und des Phenols im Vakuum wurde der Rückstand mit Essigester versetzt und mehrmals mit wäßriger Weinsäurelösung extrahiert. Die wäßrige Phase wurde mit konz. $NH_3$ oder mit verd. NaOH alkalisch gestellt und mit Essigester mehrmals extrahiert, die org. Phase mehrmals mit verd. NaOH und $H_2O$ gewaschen, über $Na_2SO_4$ getrocknet und am Rotationsverdampfer vom Lösungsmittel befreit. Die Rohprodukte wurden mit Ether aufgekocht und das Produkt abgesaugt. Zur weiteren Reinigung wurden die Produkte aus einem geeigneten Lösungsmittel umkristallisiert (s. Tabelle).

Nach dieser Vorschrift wurden die Verbindungen der Beispiele 1 bis 6 erhalten.

Beispiele 1 bis 6, Verbindungen der allgemeinen Formel I, $R^1$ = $CH_3$, $R^2$ = $R^4$ = $R^6$ = H, $R^5$ = $OCH_3$

| Beispiel | $R^3$ | Ausbeute % d.Th. | Umkristall. aus | Fp (°C) |
|---|---|---|---|---|
| 1 | 2-Thienyl | 40 | 1) Ethanol 2) Essigester | 233-234 |
| 2 | 3-Thienyl | 66 | Ethanol | 201-202 |
| 3 | 3-Methyl-2-thienyl | 70 | | 214-215 |
| 4 | 2-Furanyl | 22[a)] | | 215-216 |
| 5 | 5-Methyl-2-furanyl | 15[b)] | Ethanol | 179-180 |
| 6 | 1-Methyl-2-pyrrolyl | 10[a)b)] | Ethanol | 219-220 |

a) Reinigung durch Säulenchromatographie an Kieselgel mit $CH_2Cl_2/CH_3OH$ 95/5
b) Die Umsetzung wurde in Gegenwart von 3 Äquivalenten N-Methylmorpholin durchgeführt.

Analog können durch Umsetzung der entsprechenden 4-Chlor-, 4-Brom- oder 4-Phenoxychinoline mit den Aminen $R^3$-$CH_2$-$NHR^4$ die folgenden Verbindungen der Formel I erhalten werden:

| Beispiel 7 | | | |
|---|---|---|---|
| $R^4$ = $R^6$ = H, $R^5$ = $OCH_3$ | | | |
| Bsp. | $R^1$ | $R^2$ | $R^3$ |
| 7a | $C_2H_5$ | H | 3-Methyl-2-thienyl |
| 7b | i-$C_3H_7$ | H | 2-Thienyl |
| 7c | n-$C_4H_9$ | H | 2-Methyl-3-thienyl |
| 7d | $CH_2OH$ | H | 3-Methyl-2-thienyl |
| 7e | $CH_2OCH_3$ | H | 1-Methyl-2-pyrrolyl |
| 7f | $CH_2OCH_3$ | H | 3-Methyl-2-thienyl |
| 7g | $CH_2OCH_3$ | H | 2-Methyl-3-thienyl |
| 7h | $CH_3$ | $CH_3$ | 2-Thienyl |
| 7i | $CH_3$ | $CH_3$ | 3-Thienyl |
| 7j | $CH_3$ | $CH_3$ | 3-Methyl-2-thienyl |
| 7k | $CH_3$ | $CH_3$ | 5-Methyl-2-furanyl |
| 7l | $CH_3$ | $CH_3$ | 2-Furanyl- |
| 7m | $CH_3$ | $CH_3$ | 1-Methyl-2-pyrrolyl |
| 7n | $CH_2OCH_3$ | $CH_3$ | 3-Thienyl |
| 7o | $CH_3$ | $C_2H_5$ | 2-Thienyl |
| 7p | $CH_3$ | i-$C_3H_7$ | 2-Thienyl |
| 7q | $CH_3$ | n-$C_4H_9$ | 3-Methyl-2-thienyl |
| 7r | $CH_3$ | n-$C_6H_{13}$ | 3-Thienyl |
| 7s | H | $CH_3$ | 3-Methyl-2-thienyl |
| 7t | H | $C_2H_5$ | 2-Methyl-3-thienyl |
| 7u | H | i-$C_3H_7$ | 2-Furanyl |
| 7v | H | n-$C_4H_9$ | 2-Thienyl |
| 7w | H | n-$C_6H_{13}$ | 3-Methyl-2-thienyl |
| 7x | H | H | 3-Methyl-2-thienyl |
| 7y | H | H | 1-Methyl-2-pyrrolyl |

| Beispiel 8 | | |
|---|---|---|
| $R^1$ = $CH_3$, $R^2$ = H, $R^5$ = $CH_3O$, $R^6$ = H | | |
| Bsp. | $R^3$ | $R^4$ |
| 8a | 3-Thienyl | $CH_3$ |
| 8b | 2-Thienyl | 2-Thienylmethyl |
| 8c | 2-Methyl-3-thienyl | $CH_3$ |
| 8d | 3-Methyl-2-thienyl | 3-Methyl-2-thienyl-methyl |
| 8e | 1-Methyl-2-pyrrolyl | 1-Methyl-2-pyrrolyl-methyl |
| 8f | 1-Methyl-2-pyrrolyl | 2-Thienylmethyl |
| 8g | 1-Methyl-2-pyrrolyl | $CH_3$ |
| 8h | 3-Methyl-2-thienyl | $CH_3$ |

Beispiel 9

$R^1 = CH_3$, $R^4 = R^6 = H$, $R^5 = CH_3O$

| Bsp. | $R^2$ | $R^3$ |
|---|---|---|
| 9a | H | 4-Methyl-2-thienyl |
| 9b | H | 5-Methyl-2-thienyl |

| Bsp. | $R^2$ | $R^3$ |
|---|---|---|
| 9c | $CH_3$ | 5-Methyl-2-thienyl |
| 9d | H | 4,5-Dimethyl-2-thienyl |
| 9e | H | 4,5-Dichlor-2-thienyl |
| 9f | $CH_3$ | 2-Chlor-3-thienyl |
| 9g | H | 2-Chlor-3-thienyl |
| 9h | H | 3-Methyl-2-furanyl |
| 9i | $CH_3$ | 3-Methyl-2-furanyl |
| 9j | H | 4-Methyl-2-furanyl |
| 9k | H | 2-Methyl-4-furanyl |
| 9l | $CH_3$ | 2-Methyl-4-furanyl |
| 9m | H | 2-Methyl-3-furanyl |
| 9n | H | 2,5-Dimethyl-3-furanyl |
| 9o | $CH_3$ | 2,5-Dimethyl-3-furanyl |
| 9p | $CH_3$ | 1-Methyl-2-pyrrolyl |
| 9q | H | 1-Isopropyl-2-pyrrolyl |
| 9r | H | 1-n-Butyl-2-pyrrolyl |
| 9s | H | 1-Benzyl-2-pyrrolyl |
| 9t | H | 1-Phenyl-2-pyrrolyl |
| 9u | H | 1-(4-Chlorphenyl)-2-pyrrolyl |
| 9v | H | 1-(2-Methoxyphenyl)-2-pyrrolyl |
| 9w | H | 1,3-Dimethyl-2-pyrrolyl |
| 9x | H | 1,2-Dimethyl-3-pyrrolyl |
| 9y | H | 1-Methyl-3-pyrrolyl |
| 9z | $CH_3$ | 1-Methyl-3-pyrrolyl |
| 9za | H | 1-Isopropyl-3-pyrrolyl |
| 9zb | H | 1-Phenyl-3-pyrrolyl |
| 9zc | H | 1-Benzyl-3-pyrrolyl |
| 9zd | H | 2-Benzofuranyl |
| 9ze | H | 3-Benzofuranyl |
| 9zf | H | 3-Methyl-2-benzofuranyl |
| 9zg | H | 2-Benzothienyl |
| 9zh | H | 3-Benzothienyl |
| 9zi | H | 3-Methyl-2-benzothienyl |
| 9zj | H | 3-Indolyl |
| 9zk | H | 1-Methyl-3-indolyl |
| 9zl | $CH_3$ | 2-Benzothienyl |
| 9zm | H | 2-Methyl-3-benzothienyl |
| 9zn | H | 3-Chlor-2-benzothienyl |
| 9zo | H | 3-Brom-2-benzothienyl |
| 9zp | H | 3-Jod-2-benzothienyl |

| Bsp. | $R^2$ | $R^3$ |
|---|---|---|
| 9zq | H | 2-Brom-3-benzothienyl |
| 9zr | H | 2,4,5-Trimethyl-3-thienyl |
| 9zs | H | 3,4,5-Trimethyl-2-thienyl |
| 9zt | H | 2,4,5-Trimethyl-3-furanyl |
| 9zu | H | 3,4,5-Trimethyl-2-furanyl |
| 9zv | H | 1,2,5-Trimethyl-3-pyrrolyl |
| 9zw | H | 1,2,4-Trimethyl-3-pyrrolyl |
| 9zx | H | 1,2,4,5-Tetramethyl-3-pyrrolyl |
| 9zy | H | 1,3,4-Trimethyl-2-pyrrolyl |
| 9zz | H | 1,3,5-Trimethyl-2-pyrrolyl |

| Beispiel 10 | | | | |
|---|---|---|---|---|
| $R^1$ = $CH_3$, $R^4$ = H | | | | |
| Bsp. | $R^2$ | $R^3$ | $R^5$ | $R^6$ |
| 10a | H | 3-Methyl-2-thienyl | $OC_2H_5$ | H |
| 10b | $CH_3$ | 3-Methyl-2-thienyl | $OC_2H_5$ | H |
| 10c | H | 3-Methyl-2-thienyl | O-n$C_4H_9$ | H |
| 10d | H | 2-Thienyl | O-n$C_6H_{13}$ | H |
| 10e | H | 1-Methyl-2-pyrollyl | $OCH_2C_6H_5$ | H |
| 10f | $CH_3$ | 1-Methyl-2-pyrollyl | $OCH_2C_6H_5$ | H |
| 10g | H | 5-Methyl-2-furanyl | $OCH_2C_6H_5$ | H |
| 10h | $CH_3$ | 3-Methyl-2-thienyl | $OCH_2C_6H_5$ | H |
| 10i | H | 3-Methyl-2-thienyl | $OCH_2C_6H_5$ | H |
| 10j | H | 3-Thienyl | $SCH_3$ | H |
| 10k | H | 3-Methyl-2-thienyl | $N(CH_3)_2$ | H |
| 10l | H | 3-Methyl-2-thienyl | $NHCH_2C_6H_5$ | H |
| 10m | H | 3-Methyl-2-thienyl | $NHCOC_6H_5$ | H |
| 10n | H | 2-Thienyl | $CF_3$ | H |
| 10o | H | 3-Methyl-2-thienyl | Cl | H |
| 10p | $CH_3$ | 3-Methyl-2-thienyl | Cl | H |
| 10q | H | 1-Methyl-2-pyrrolyl | Br | H |
| 10r | H | 3-Methyl-2-thienyl | $OCH_3$ | 6-$OCH_3$ |
| 10s | H | 3-Methyl-2-thienyl | $OCH_3$ | 5-$OCH_3$ |
| 10t | H | 2-Methyl-3-thienyl | $OCH_3$ | 5-$OCH_3$ |
| 10u | H | 3-Methyl-2-thienyl | $OCH_3$ | 6-Cl |
| 10v | H | 3-Methyl-2-thienyl | $OCH_3$ | 5-$CH_3$ |
| 10w | H | 3-Methyl-2-thienyl | $OCH_3$ | 6-$CH_3$ |
| 10x | H | 3-Methyl-2-thienyl | $OCH_3$ | 7-$CH_3$ |
| 10y | H | 2-Methyl-3-thienyl | $OCH_3$ | 5-$CH_3$ |
| 10z | H | 2-Methyl-3-thienyl | $OCH_3$ | 6-$CH_3$ |

| Beispiel 11 | |
|---|---|
| $R^1 = CH_3$, $R^2 = R^4 = R^6 = H$, $R^5 = OCH_3$ | |
| Bsp. | $R^3$ |
| 11a | 3-Ethyl-2-thienyl |
| 11b | 3-Isopropyl-2-thienyl |
| 11c | 3-Cyclopropyl-2-thienyl |
| 11d | 5-Ethyl-2-thienyl |
| 11e | 5-Isopropyl-2-thienyl |
| 11f | 5-tert.-Butyl-2-thienyl |
| 11g | 3,5-Dimethyl-2-thienyl |
| 11h | 3,4-Dimethyl-2-thienyl |
| 11i | 3,4,5-Trimethyl-2-thienyl |
| 11j | 2-Methyl-3-thienyl |
| 11k | 4-Methyl-3-thienyl |
| 11l | 5-Methyl-3-thienyl |
| 11m | 2,4-Dimethyl-3-thienyl |
| 11n | 2,5-Dimethyl-3-thienyl |
| 11o | 4,5-Dimethyl-3-thienyl |
| 11p | 2,4,5-Trimethyl-3-thienyl |

| Beispiel 12 | |
|---|---|
| $R^1 = CH_3$, $R^2 = R^4 = R^6 = H$, $R^5 = OCH_3$ | |
| Bsp. | $R^3$ |
| 12a | 3-Chlor-2-thienyl |
| 12b | 4-Chlor-2-thienyl |
| 12c | 5-Chlor-2-thienyl |
| 12d | 3,4-Dichlor-2-thienyl |
| 12e | 3,4,5-Trichlor-2-thienyl |
| 12f | 5-Chlor-3-methyl-thienyl |
| 12g | 2-Chlor-3-thienyl |
| 12h | 4-Chlor-3-thienyl |
| 12i | 5-Chlor-3-thienyl |
| 12j | 2,5-Dichlor-3-thienyl |
| 12k | 4,5-Dichlor-3-thienyl |
| 12l | 4-Chlor-2,5-dimethyl-2-thienyl |
| 12m | 2-Chlor-5-methyl-2-thienyl |
| 12n | 3-Brom-2-thienyl |
| 12o | 4-Brom-2-thienyl |
| 12p | 5-Brom-2-thienyl |
| 12q | 3,4-Dibrom-2-thienyl |
| 12r | 3,5-Dibrom-2-thienyl |
| 12s | 4,5-Dibrom-2-thienyl |
| 12t | 3-Brom-5-methyl-2-thienyl |
| 12u | 4-Brom-3-methyl-2-thienyl |
| 12v | 5-Brom-3-methyl-2-thienyl |
| 12w | 2-Brom-3-thienyl |
| 12x | 4-Brom-3-thienyl |
| 12y | 5-Brom-3-thienyl |
| 12z | 2,4-Dibrom-3-thienyl |
| 12za | 2,5-Dibrom-3-thienyl |
| 12zb | 4,5-Dibrom-3-thienyl |
| 12zd | 2,4,5-Tribrom-3-thienyl |
| 12ze | 5-Brom-2-methyl-3-thienyl |
| 12zf | 2-Brom-5-methyl-3-thienyl |
| 12zg | 4-Brom-2-methyl-3-thienyl |
| 12zh | 2-Brom-4-methyl-3-thienyl |
| 12zi | 3-Jod-2-thienyl |
| 12zj | 4-Jod-2-thienyl |
| 12zk | 5-Jod-2-thienyl |
| 12zl | 3-Jod-5-methyl-2-thienyl |
| 12zm | 5-Jod-3-methyl-2-thienyl |
| 12zn | 2-Jod-3-thienyl |
| 12zo | 5-Jod-3-thienyl |
| 12zp | 2-Jod-4-methyl-3-thienyl |
| 12zq | 4-Jod-2-methyl-3-thienyl |
| 12zr | 5-Jod-2-methyl-3-thienyl |
| 12zs | 2,5-Dimethyl-4-Jod-3-thienyl |

| Beispiel 13 | | |
|---|---|---|
| $R^1$ = $CH_3$, $R^2$ = $R^4$ = $R^6$ = H, | | |
| Bsp. | $R^3$ | $R^5$ |
| 13a | 3-Methyl-2-thienyl | $NHCOCH_3$ |
| 13b | 3-Methyl-2-thienyl | $O\text{-}iC_3H_7$ |
| 13c | 3-Methyl-2-thienyl | $O\text{-}nC_3H_7$ |
| 13d | 3-Methyl-2-thienyl | $NHCH_3$ |
| 13e | 3-Methyl-2-thienyl | $COCH_3$ |
| 13f | 3-Methyl-2-thienyl | COOH |
| 13g | 3-Methyl-2-thienyl | $COOC_2H_5$ |
| 13h | 3-Methyl-2-thienyl | $CONH_2$ |
| 13i | 3-Methyl-2-thienyl | $CON(CH_3)_2$ |
| 13j | 3-Methyl-2-thienyl | $CH_2OH$ |
| 13k | 2-Methyl-3-thienyl | $CH_2OH$ |
| 13l | 1-Methyl-2-pyrrolyl | $CH_2OH$ |
| 13m | 3-Methyl-2-thienyl | $CH(OH)CH_3$ |
| 13n | 2-Methyl-3-thienyl | $CH(OH)CH_3$ |
| 13o | 2-Thienyl | $CH_3$ |
| 13p | 3-Thienyl | $CH_3$ |
| 13q | 3-Methyl-2-thienyl | $CH_3$ |
| 13r | 2-Methyl-3-thienyl | $CH_3$ |
| 13s | 1-Methyl-2-pyrrolyl | $CH_3$ |
| 13t | 1-Isopropyl-2-pyrrolyl | $CH_3$ |
| 13u | 1,2,5-Trimethyl-3-pyrrolyl | $CH_3$ |
| 13v | 3-Methyl-2-thienyl | $C_2H_5$ |
| 13w | 2-Methyl-3-thienyl | $C_2H_5$ |
| 13x | 3-Methyl-2-thienyl | $nC_3H_7$ |
| 13y | 3-Methyl-2-thienyl | $iC_3H_7$ |
| 13z | 3-Methyl-2-thienyl | $CH_2OCH_3$ |
| 13za | 3-Methyl-2-thienyl | $CH_2OC_2H_5$ |
| 13zb | 3-Methyl-2-thienyl | $CH_2OiC_3H_7$ |

## Ansprüche

1. Verbindungen der allgemeinen Formel I

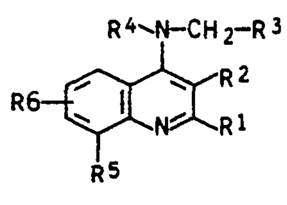

(I),

in der
$R^1$ ein Wasserstoffatom oder eine $C_1$-$C_4$-Alkylgruppe, die durch eine Hydroxi- oder eine $C_1$-$C_4$-Alkoxigruppe substituiert sein kann,
$R^2$ ein Wasserstoffatom oder eine $C_1$-$C_6$-Alkylgruppe,
$R^3$ einen Thienylrest, der durch 1 bis 3 $C_1$-$C_4$-Alkylgruppen und/oder 1 bis 2 Halogenatome substituiert sein kann,
einen Furanylrest, der durch 1 bis 3 $C_1$-$C_4$-Alkylgruppen substituiert sein kann,
einen Pyrrolylrest, der am Pyrrol-Stickstoffatom ein Wasserstoff, eine $C_1$-$C_6$-Alkylgruppe, einen Phenyl($C_1$-$C_3$-alkyl)rest oder einen Phenylrest tragen kann und an den Kohlenstoffatomen des Pyrrol-Ringes mit 1 bis 3 $C_1$-$C_4$-Alkylgruppen substituiert sein kann,

wobei der Thienyl-, Furanyl- oder Pyrrolylrest auch benzokondensiert sein kann,
$R^4$ ein Wasserstoffatom, eine $C_1$-$C_4$-Alkylgruppe oder den Rest -$CH_2$-$R^3$, wobei R3 obige Bedeutung besitzt,
$R^5$ eine $C_1$-$C_6$-Alkylgruppe, die durch eine Hydroxi- oder $C_1$-$C_3$-Alkoxigruppe substituiert sein kann, eine Hydroxi-, $C_1$-$C_6$-Alkoxi-, Phenoxi-, Phenyl-($C_1$-$C_3$-alkoxi)-, $C_1$-$C_6$-Alkylthio-, Phenylthio-, Phenyl-($C_1$-$C_3$-alkylthio)-, Amino-, $C_1$-$C_4$-Alkylamino-, Di-($C_1$-$C_4$-alkyl)amino-, Phenylamino-, N-phenyl-N-($C_1$-$C_4$-alkyl)-amino-, (Phenyl-($C_1$-$C_4$-alkyl))-amino-, N-(Phenyl-($C_1$-$C_4$-alkyl))-N-($C_1$-$C_4$-alkyl)-amino-, $C_1$-$C_4$-Alkanoylamino-, Benzoylamino-, (Phenyl-($C_1$-$C_3$-alkanoyl))amino-, $C_1$-$C_6$-Alkanoyl-, Hydroxycarbonyl-, $C_1$-$C_6$-Alkoxicarbonyl-, Aminocarbonyl-, $C_1$-$C_6$-Alkylaminocarbonyl-, Di-($C_1$-$C_6$-Alkyl)aminocarbonyl- oder Trifluormethylgruppe oder ein Halogenatom und
$R^6$ ein Wasserstoffatom, eine $C_1$-$C_4$-Alkyl- oder $C_1$-$C_4$-Alkoxygruppe oder ein Halogenatom bedeuten, sowie deren physiologisch verträgliche Salze.

2. Verbindungen gemäß Anspruch 1, worin $R^2$ ein Wasserstoffatom bedeutet, sowie deren physiologisch verträgliche Salze.

3. Verbindungen gemäß Anspruch 2, worin $R^1$ eine ggf. durch eine Hydroxi-oder $C_1$-$C_3$-Alkoxigruppe substituierte $C_1$-$C_3$-Alkylgruppe, $R^3$ einen gegebenenfalls durch 1 bis 3 $C_1$-$C_3$-Alkylgruppen und/oder 1 bis 2 Halogenatome substituierten Thienylrest oder einen ggf. durch 1 bis 3 $C_1$-$C_3$-Alkylgruppen substituierten Furanylrest oder einen Pyrrolrest, der am Pyrrol-Stickstoffatom durch eine $C_1$-$C_4$-Alkyl-, eine Benzyl-oder Phenylgruppe substituiert ist und an den Kohlenstoffatomen des Pyrrolringes mit 1 bis 3 Methylgruppen substituiert sein kann, $R^4$ ein Wasserstoffatom oder eine Methylgruppe, $R^5$ eine $C_1$-$C_3$-Alkylgruppe, die durch eine Hydroxi- oder Methoxigruppe substituiert sein kann, die Hydroxi- oder eine $C_1$-$C_3$-Alkoxigruppe und $R^6$ ein Wasserstoffatom bedeuten, sowie deren physiologisch verträgliche Salze.

4. Verbindungen gemäß Anspruch 1, worin $R^2$ eine $C_1$-$C_3$-Alkylgruppe bedeutet, sowie deren physiologisch verträgliche Salze.

5. Verfahren zur Herstellung der Verbindungen der Formel I

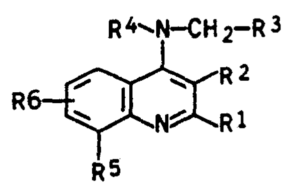

(I),

nach Anspruch 1, dadurch gekennzeichnet, daß ein Chinolin der Formel II

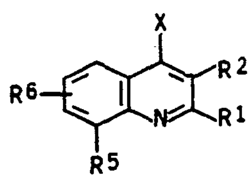

(II),

worin X eine nukleofuge Abgangsgruppe darstellt und $R^1$, $R^2$, $R^5$ und $R^6$ die in Anspruch 1 genannten Bedeutungen besitzen, mit einem Amin der Formel $R^2CH_2NHR^4$ in Gegenwart oder Abwesenheit eines Lösungsmittels bei einer Temperatur im Bereich von 50 bis 250°C umgesetzt wird.

6. Verwendung einer Verbindung der Formel I nach einem der Ansprüche 1 bis 4 zur Herstellung eines Arzneimittels.

7. Verwendung einer Verbindung der allgemeinen Formel I nach einem der Ansprüche 1 bis 4 zur Herstellung eines Arzneimittels gegen Krankheiten, bei denen eine Herabsetzung der Magensäuresekretion die Heilung günstig beeinflußt.

8. Arzneimittel zur oralen oder rektalen Applikation, das als Wirkstoff pro Einzeldosis 10 bis 1000 mg einer Verbindung der Formel I nach einem der Ansprüche 1 bis 4 neben üblichen galenischen Hilfsmitteln enthält.

9. Arzneimittel zur intravenösen Applikation, das als Wirkstoff 0,01 bis 1,0 mg/kg Körpergewicht einer Verbindung der Formel I nach einem der Ansprüche 1 bis 4 neben üblichen galenischen Hilfsmitteln enthält.

10. Arzneimittel gegen Krankheiten, bei denen eine Herabsetzung der Magensäuresekretion die Heilung günstig beeinflußt, das als Wirkstoff eine wirksame Menge einer Verbindung der Formel I nach einem der

Ansprüche 1 bis 4 neben üblichen galenischen Hilfsmitteln enthält.

Patentansprüche für folgende Vertragsstaaten: GR, ES

1. Verfahren zur Herstellung von Verbindungen der allgemeinen Formel I

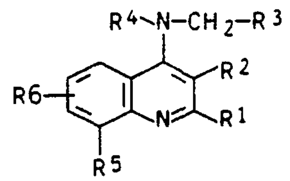

(I),

worin

$R^1$ ein Wasserstoffatom oder eine $C_1$-$C_4$-Alkylgruppe, die durch eine Hydroxi- oder eine $C_1$-$C_4$-Alkoxigruppe substituiert sein kann,

$R^2$ ein Wasserstoffatom oder eine $C_1$-$C_6$-Alkylgruppe,

$R^3$ einen Thienylrest, der durch 1 bis 3 $C_1$-$C_4$-Alkylgruppen und/oder 1 bis 2 Halogenatome substituiert sein kann,

einen Furanylrest, der durch 1 bis 3 $C_1$-$C_4$-Alkylgruppen substituiert sein kann,

einen Pyrrolylrest, der am Pyrrol-Stickstoffatom ein Wasserstoff, eine $C_1$-$C_6$-Alkylgruppe, einen phenyl($C_1$-$C_3$-alkyl)rest oder einen Phenylrest tragen kann und an den Kohlenstoffatomen des Pyrrol-Ringes mit 1 bis 3 $C_1$-$C_4$-Alkylgruppen substituiert sein kann,

wobei der Thienyl-, Furanyl- oder Pyrrolylrest auch benzo-kondensiert sein kann,

$R^4$ ein Wasserstoffatom, eine $C_1$-$C_4$-Alkylgruppe oder den Rest -$CH_2$-$R^3$, wobei $R^3$ obige Bedeutung besitzt,

$R^5$ eine $C_1$-$C_6$-Alkylgruppe, die durch eine Hydroxi- oder $C_1$-$C_3$-Alkoxigruppe substituiert sein kann, eine Hydroxi-, $C_1$-$C_6$-Alkoxi-, Phenoxi-, Phenyl-($C_1$-$C_3$-alkoxi)-, $C_1$-$C_6$-Alkylthio-, Phenylthio-, Phenyl-($C_1$-$C_3$-alkylthio)-, Amino-, $C_1$-$C_4$-Alkylamino-, Di-($C_1$-$C_4$-alkyl)amino-, Phenylamino-, N-Phenyl-N-($C_1$-$C_4$-alkyl)-amino-, (Phenyl-($C_1$-$C_4$-alkyl))-amino, N-(Phenyl-($C_1$-$C_4$-alkyl))-N-($C_1$-$C_4$-alkyl)-amino-, $C_1$-$C_4$-Alkanoyl amino-, Benzoylamino-, (Phenyl-($C_1$-$C_3$-alkanoyl))amino-, $C_1$-$C_6$-Alkanoyl-, Hydroxycarbonyl-, $C_1$-$C_6$-Alkoxicarbonyl-, Aminocarbonyl-$C_1$-$C_6$-Alkylaminocarbonyl-, Di-($C_1$-$C_6$-Alkyl)aminocarbonyl oder Trifluormethylgruppe oder ein Halogenatom und

$R^6$ ein Wasserstoffatom, eine $C_1$-$C_4$-Alkyl- oder $C_1$-$C_4$-Alkoxygruppe oder ein Halogenatom bedeuten, bedeutet, dadurch gekennzeichnet, daß ein Chinolin der Formel II

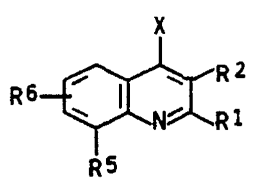

(II),

worin X eine nukleofuge Abgangsgruppe darstellt und $R^1$, $R^2$, $R^5$ und $R^6$ die obigen Bedeutungen besitzen, mit einem Amin der Formel $R^2CH_2NHR^4$ bei einer Temperatur im Bereich von 50 bis 250°C umgesetzt wird.

2. Verwendung einer nach Anspruch 1 erhältlichen Verbindung der Formel I zur Herstellung eines Arzneimittels.

3. Verwendung einer nach Anspruch 1 erhältlichen Verbindung der Formel I zur Herstellung eines Arzneimittels gegen Krankheiten, bei denen eine Herabsetzung der Magensäuresekretion die Heilung günstig beeinflußt.